# EUROPÄISCHE PATENTSCHRIFT

(11) **EP 2 438 900 B1**
(45) Veröffentlichungstag und Bekanntmachung des Hinweises auf die Patenterteilung: **22.04.2015**
(21) Anmeldenummer: 11180663.4
(22) Anmeldetag: 09.09.2011
(51) Int. Cl.: A61Q 5/00, A61Q 5/12, A61K 8/27, A61K 8/97, A61K 8/81, A61Q 5/06, A61K 8/19

(54) **Pflegende Pflanzenhaarfarbe**
Protective plant hair dye
Colorant capillaire de soin à base de plantes

(30) Priorität: 05.10.2010 DE 102010041974
(43) Veröffentlichungstag der Anmeldung: 11.04.2012
(73) Patentinhaber: Henkel AG & Co. KGaA, 40589 Düsseldorf (DE)
(72) Erfinder: Goutsis, Konstantin, 41812 Erkelenz (DE); Janßen, Frank, 41470 Neuss (DE)

(56) Entgegenhaltungen:
- WO-A1-2005/094763
- DE-A1- 4 341 647
- JP-A- 2003 026 552
- US-A- 3 958 581
- US-A1- 2002 124 859
- US-A1- 2009 249 563
- DATABASE WPI Week 199216 30. April 1992 (1992-04-30) Thomson Scientific, London, GB; AN 1992-127243 XP002669288, "Non-irritant hair dyeing compsn. - contg. sodium and/or potassium copper chlorophyllin and vegetable extract contg. tannin", -& JP 4 069324 A (YAMAHATSU SANGYO KAISHA) 4. März 1992 (1992-03-04)

## Beschreibung

Die Erfindung betrifft Mittel zur Färbung keratinhaltiger Fasern, insbesondere menschlicher Haare, enthaltend natürliche Farbstoffe, bestimmte Metallsalze sowie bestimmte Pflegepolymere, sowie deren Verwendung zur Färbung keratinhaltiger Fasern und Färbeverfahren, in dem diese Mittel zur Anwendung kommen.

Zur Bereitstellung farbverändernder kosmetischer Mittel, insbesondere für die Haut oder keratinhaltige Fasern, wie beispielsweise menschliche Haare, kennt der Fachmann je nach Anforderungen an die Färbung diverse Färbesysteme.

Für die Färbung von Haaren besteht beim Kunden neben Färbesystemen mit synthetischen Farbstoffen, wie Oxidationsfarbstoffvorprodukten und direktziehenden Farbstoffen, ein verstärktes Interesse nach natürlichen Färbesystemen. Solche natürliche Färbesysteme umfassen beispielsweise Henna, welches seit Jahrhunderten für die kosmetische Farbveränderung von Haut und Haar eingesetzt wird. Als nachteilig bei solchen natürlichen Färbesystemen erweist die üblicherweise wenig ausgeprägte Farbintensität und unzureichende Haltbarkeit der Färbung gegenüber äußeren Einflüssen, insbesondere im Hinblick auf Waschechtheit.

Oxidative Färbesysteme erzielen zwar deutlich verbesserte Echtheitseigenschaften, sind jedoch nachteilig im Hinblick auf Haarschädigung durch vorhandene Alkalisierungsmittel und Oxidationsmittel. Die Schädigung bzw. Pflegeleistung von Haarbehandlungsmitteln lässt sich in vielfältiger Weise bestimmen. Dazu gehören Methoden, die auf die Schädigung der inneren Struktur der Haare gerichtet sind, wie NIR-Bestimmungen des Cysteinsäureanteils im Keratin oder DSC-Schmelzpunkt-Messungen, ebenso wie Methoden, die auf die Haaroberfläche abzielen, wie Kämmbarkeit.

Aus WO2007/130777A1 sind Färbesysteme bekannt, die eine Verbesserung der Farbintensität und der Waschechtheiten durch Kombination von natürlichen Farbstoffen mit Metallsalzen erreichen. Es zeigte sich jedoch, dass die so gefärbten Haare Defizite bezüglich ihres Pflegezustands, insbesondere der Haptik der Haare und der Kämmbarkeit, ausweisen.

Aufgabe der vorliegenden Anmeldung war daher die Bereitstellung von Färbezubereitungen auf Basis von natürlichen Farbstoffen, die neben sehr guter Echtheit der Färbungen und kräftiger, intensiver Färbeleistung auch einen verbesserten Pflegezustand der Haare ermöglichen.

DE 43 41 647 A1 offenbart Pflanzenhaarfarbenzubereitungen, die pflegende Eigenschaften, wie Haargriff- und Kämmbarkeitsverbesserung, aufweisen (siehe Spalte 1, Zeile 67-Spalte 2, Zeile 3). Die Zubereitungen aus DE 43 41 647 A1 bestehen aus Polyquaternium 10 (0,01 bis 3 - vorzugsweise 0,1 bis 2 - Gewichtsprozent ) und haarfärbenden Pflanzenbestandteilen (1 bis 50 - vorzugsweise 10 bis 40 - Gewichtsprozent) (siehe Spalte 2, Zeile 22-28). Im Beispiel 1, 50g einer Mischung aus 70% *Indigoferae,* 20% *Henna* und 10% Polyquaternium-10 werden mit 300 g Wasser verdünnt bzw. angerührt und auf das Haar gebracht. Es resultiert ein brauner Farbton.

Es wurde nun überraschend gefunden, dass insbesondere der Zusatz von bestimmten kationischen oder amphoteren Polymeren zu Färbezubereitungen auf Basis von natürlichen Farbstoffen zu einer signifikanten Verbesserung der Kämmbarkeit der gefärbten Haare führt.

Ein erster Gegenstand der Erfindung ist daher ein Mittel zum Färben menschlicher Haare, welches in einem kosmetisch verträglichen Träger mindestens einen natürlichen Farbstoff, ausgewählt aus Alkaloid-Farbstoffen, Anthrachinon-Farbstoffen, Betalainen, Carotinoiden, Cathechinen, Curcumidoiden, Flavinen, Flavonoiden, indigoiden Farbstoffen, Naphthochinonen, und weiteren phenolischen Farbstoffen sowie mindestens ein zweiwertiges Metall-Salz enthält, und welches dadurch gekennzeichnet ist, dass das Mittel weiterhin mindestens ein amphoteres Polymer enthält.

Unter einem kosmetisch verträglichen Träger ist im Sinne der vorliegenden Erfindung eine flüssige Zubereitung zu verstehen, welche geeignet ist, die Wirkstoffe unter den Lagerbedingungen zu stabilisieren. Der kosmetisch verträgliche Träger ist dabei bevorzugt wässrig, alkoholisch oder wässrig-alkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Ein wässriger Träger enthält im Sinne der Erfindung mindestens 30 Gew.-%, insbesondere mindestens 50 Gew.-% Wasser, bezogen auf das Gesamtgewicht der Komponente. Unter alkoholischen Trägern sind im Sinne der vorliegenden Erfindung Zusammensetzungen, enthaltend mindestens 50 Gew.-% eines C₁-C₄-Alkohols, bezogen auf das Gesamtgewicht der Komponente, insbesondere Ethanol bzw. Isopropanol, zu verstehen. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Komponente, insbesondere Ethanol bzw. Isopropanol, zu verstehen. Die erfindungsgemäßen Komponenten können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.

Als ersten wesentlichen Inhaltsstoff enthält das Mittel des ersten Erfindungsgegenstands mindestens einen natürlichen Farbstoff, welcher ausgewählt ist aus Alkaloid-Farbstoffen, Anthrachinon-Farbstoffen, Betalainen, Carotinoiden, Cathechinen, Curcumidoiden, Flavinen, Flavonoiden, indigoiden Farbstoffen, Naphthochinonen und weiteren phenolischen Farbstoffen.

Als natürliche Farbstoffe im Sinne der Erfindung sind solche Farbstoffe zu verstehen, die aus natürlichen Quellen, insbesondere Pflanzenteilen, gewonnen werden. Dazu eignen sich unterschiedliche Pflanzenteile, je nach gewünschtem Farbstoff. Es versteht sich jedoch, dass ein Farbstoff aus mehr als einem Pflanzenteil gewonnen werden kann. Die bevorzugten Pflanzenteile zur Gewinnung der natürlichen Farbstoffe sind Wurzel, Stängel, Rinde, Kernholz, Harz, Blüte, Blatt, Frucht und/oder Pflanzensaft.

Im Sinne der Erfindung sind natürliche Farbstoffe Verbindungen, die entweder Farbstoffe als solche darstellen, oder die gegebenenfalls auch Farbstoffvorstufen darstellen, die durch nachfolgende chemische Reaktionen den eigentlichen Farbstoff ausbilden. Genannt sei hier insbesondere die Oxidation mit Luftsauerstoff zur Ausbildung des Chromophors.

Es ist insbesondere im Sinne der Erfindung nicht erforderlich, dass die natürlichen Farbstoffe jeweils einheitliche Verbindungen darstellen. Bedingt durch unterschiedliche, nicht einheitliche Gewinnungsverfahren wie beispielsweise unterschiedliche Extraktions- oder Fermentationsbedingungen oder durch Nutzung verschiedener, regionaler Unterarten und verschiedener Pflanzenteile oder teilweise auch aus kultur-historischer Tradition enthalten manche genannten natürliche Farbstoffe bewusst keine einheitlichen Verbindungen, sondern Gemische aus Farbstoffen, Farbstoffvorstufen und weiteren Komponenten, wie insbesondere Gerbstoffen. Es kann daher erfindungsgemäß vorteilhaft sein, anstelle einer einheitlichen Verbindung als Farbstoff direkt einen Extrakt aus natürlichem Ursprung einzusetzen. Dazu eignen sich beispielsweise Extrakte aus Pflanzen, wie Emblica, Alfalfa, Tee und Sandelholz. Gleichfalls eignen sich Extrakte der nachstehend angeführten Pflanzen.

Alkaloid-Farbstoffe gemäß vorliegender Erfindung sind beispielsweise Berberin oder Awobamin.

Anthrachinon-Farbstoffen gemäß vorliegender Erfindung sind beispielsweise Purpurin (C.I. 75410), Pseudopurpurin (C.I. 75420; C.I. 58220-snythetisch), Alizarin (C.I. 75330), Rubiadin (C.I. 75350) und Munjistin (C.I. 75370) aus Krapp-Wurzeln (C.I. Natural Red 8) *- Rubia peregrina* und *Rubia tinctorum;* Purpurin (C.I. 75410), Pseudopurpurin (C.I. 75420; C.I. 58220-snythetisch), Alizarin (C.I. 75330), und Munjistin (C.I. 75370) sowie Nordamnacanthal, Physcion und Purpuroxanthin (C.I. 75340)
aus den Wurzeln und Stängeln des Indischen Krapp (C.I. Natural Red *1*6) *- Rubia cordifolia* und *Rubia sikkimensis;*
Emodin, Chrystophanol (C.I. 75400; C.I. Natural Yellow 23), Aloe-emodin, Physcion, Rhein, den Heterodianthronen Rheidin A, B, C, Palmidin A, B, C, D und Sennidin C sowie davon abgeleiteten Anthra- und Diglycosiden aus Rharbarber-Wurzeln - *Rheum undulatum L., Rheum palmatum L.* und weiteren *Rheum*-Spezies;
Purpurin (C.I. 75410), Pseudopurpurin (C.I. 75420; C.I. 58220-snythetisch), Alizarin (C.I. 75330), Rubiadin (C.I. 75350), Purpuroxanthin (C.I. 75340), Lucidin, Alizarin-1-methylether, Alizarin-2-methylether, 2-Hydroxyanthrachinon und 1-Hydroxy-2-methylanthrachinon aus den Wurzeln des Echten Labkraut - *Galium verum L.,* Gemeinen Labkraut - *Galium mollugo L.,* Klebkraut - *Galium aparine L.,* Waldmeister- *Galium odoratum* (C.I. Natural Red 14);
Purpurin (C.I. 75410), Pseudopurpurin (C.I*.* 75420; C.I. 58220-snythetisch) und Alizarin (C.I. 75330) aus Färbermeisterwurzel (C.I. Natural Red *13*) *- Asperula tinctoria;* Emodin, Emodin-Glucoside wie Glucofragulin A, Glucofrangulin B, Frangulin A, Frangulin B, Physcion und Chrysophanol (C.I. 75400) aus der Rinde des Faulbaum - *Rhamnus fragula L. tinctoria.* Weitere geeignete Anthrachinon-Farbstoffe sind Morindanigrin, Munjistin, Morindadiol und Morindon.

Betalaine gemäß vorliegender Anmeldung sind beispielsweise Betanidin und Praebetanin aus Rote Beete Wurzeln - *Beta vulgaris.*

Carotinoide gemäß vorliegender Erfindung sind beispielsweise beta-Carotin, Crocetin, Bixin, Canthaxanthin, Lycopin, Capsanthin, Apocarotinal sowie Xanthophylle als Hydroxy-, Epoxy- oder Oxo-Derivate solcher Carotinoid-Farbstoffe.

Cathechine gemäß vorliegender Erfindung sind beispielsweise Catechin, Theaflavin und Isotheaflavin aus Catechu (C.I. Natural Brown 3), einer Mischung aus Extrakten von Färbepflanzen, vorzugsweise *Acacia catechu, Areca catechu* und *Uncaria gambier,* sowie aus den Blättern von grünem und schwarzem (= fermentiertem grünen) Tee - *Camellia sinensis.*

Curcumidoide gemäß vorliegender Erfindung sind Diaryloylmethanfarbstoffe, wie sie beispielsweise aus der Wurzel von Curcuma (Gelbwurzel; C.I. Natural Yellow 3) - *Curcuma domestica* mit den färbenden Inhaltsstoffen Curcumin (C.I. 75300), Demethoxycurcumin und Bisdemethoxycurcumin erhältlich sind.

Ein Flavin gemäß vorliegender Erfindung ist beispielsweise Riboflavin.

Flavonoide gemäß vorliegender Erfindung sind beispielsweise aus den Blütenköpfen von Römischer Kamille - *Chamaemelum nobile* (C.I. Natural Yellow 1) und Deutscher Kamille - *Chamomilla recutica* mit den färbenden Inhaltsstoffen Apigenin (C.I. 75580; C.I. Natural Yellow 1,2), Rutin (C.I. 75730; Natural Yellow 10), sowie untergeordnet Luteolin (C.I. 75590), Kämpferol (C.I. 75460, C.I. Natural Yellow 13, 10) und Quercetin (C.I. 75670; C.I. Natural Yellow 10,13; C.I. Natural Red 1) und davon abgeleiteten Glucosiden;
aus den Blättern und Stengeln von Salbei - *Salvia tribola L.* mit den färbenden Inhaltsstoffen Luteolin (C.I. 75590) und Salvigenin;
aus den Blättern und Stengeln von Rosmarin - *Rosmarinus Officinalis* mit den färbenden Inhaltsstoffen Luteolin (C.I. 75590) und Apigenin (C.I. 75580; C.I. Natural Yellow 1,2) sowie weiteren Flavonoiden erhältlich. Weitere geeignete Flavonoide sind beispielsweise Datiscetin, Morin, Fisetin, Gossypetin, Pratol, Fulsugetin, Genistein (Prunetol), Sophoretin, Isorhamnetin, Xanthorhamnin,
Rhamnazin, Quercimeritrin, Quercitrin, das Chalkon Butein,sowie Anthocyane, wie beispielsweise Dracorhodin.

Zu den Flavonoiden sind weiterhin Neoflavanoidfarbstoffe zu zählen, die beispielsweise aus Blauholz (C.I. Natural Black 1), Kernholz von *Haematoxylum campechianum L.* mit dem Farbstoffvorprodukt Hämatoxylin, welches oxidativ in den Farbstoff Hämatein (C.I. 75290, C.I. Natural Black 1 und 2) umgewandelt wird;
aus Sappanholz - *Caesalpinia sappan L.,* Brazilienholz - *Caesalpinia brasiliensis L.,* Nicaraguaholz - *Caesalpinia echinata,* Pernambunkholz - *Caesalpinia crista L.,* Brasilietteholz - *Haematoxylum braziletto,* jeweils als Kernholz (C.I. Natural Red 24), mit dem Farbstoffvorprodukt Brasilin, welches oxidativ in den Farbstoff Brasilein (C.I. 75280, C.I. Natural Red 24) umgewandelt wird, erhältlich sind.

Zu den erfindungsgemäß einsetzbaren Flavonoiden zählen weiterhin ,unlösliche Rotholz'-Farbstoffe, wie beispielsweise aus Rotem Sandelholz, Kernholz von *Pterocarpus santalinus L.,* Narraholz - *Pterocarpus indicus L.,* Barholz - *Pterocarpus soyauxü L.,* Camholz - *Bahia nitida* (C.I. Natural Red 22) mit den färbenden Inhaltsstoffen Santalin A, Santalin B, Tetra-O-methylsantarubin, Hompterocarpin, Pterocarpin, Maackiain, den Isoflavioniden Formononetin und Santal, sowie dem Chalkon Isoliquiritigenin und dem Flavon Liquiritigenin.

Indigoide Farbstoffe gemäß vorliegender Anmeldung sind wie beispielsweise aus den Blättern und teilweise Stängeln des Indischem Indigo - *Indigofera tinctoria* (*Indigofera argentea*), Färberwaid - *Isatis tinctoria,* Färberoleander - *Wrightia tinctoria* und Wildem Indigo - *Baptisia tinctoria* (C.I. Natural Blue 1) mit den Farbstoffvorprodukten Isatan B und Indican, welche durch einen Gärungsprozess oxidativ in den eigentlichen Farbstoff Indigo (C.I. 75780; C.I. 73000) sowie untergeordnet Indirubin (C.I. 75790; C.I. 73200) und Isatin überführt werden und beispielsweise unter den Namen Indigofera folium oder Henna, schwarz als Handelsprodukt, zumeist in Kombination mit Extrakten von *Lawsonia Inermis,* erhältlich.

Naphthochinone bzw. Naphthochinon-Farbstoffe gemäß vorliegender Erfindung sind beispielsweise enthalten in Henna, rot (C.I. Natural Orange 6) aus den Blättern des Hennastrauchs - *Lawsonia inermis L.* mit dem färbenden Inhaltsstoff Lawson (C.I. 75480),

in Fruchtschalen und Blättern (C.I. Natural Brown 7) aus dem Walnussbaum - *Juglus regia L.* mit dem färbenden Inhaltsstoff Juglon (C.I. 75500),

in Alkannawurzel oder Shikoninwurzel (C.I. Natural Red *20) - Alkanna tinctoria* beziehungsweise *Lithospermum erythrorhizon* mit dem färbenden Inhaltsstoffen Alkannan (C.I. 75520) und Alkannin (C.I. 75530) beziehungsweise Shikonin (C.I. 75535),

in Lapachoholz von *Tecoma ipé, Tecoma lapacho, Tecoma leucoxylon* oder *Tecoma achracea* (C.I. Natural Yellow 16) mit dem färbenden Inhaltsstoffen Lapachol (C.I. 75490), sowie untergeordnet Desoxylapachol, α-(alpha)-Lapachon, β-(beta)-Lapachon, Melchinon I, Lapachonon und Dehydro-α-Iapachon und Deoxysantalin.

Zu den weiteren phenolischen Farbstoffen gemäß vorliegender Erfindung sind beispielsweise Atromentin, Rottlerin sowie Gallotanninfarbstoffe, wie beispielsweise aus der Rinde von Stiel- oder Sommereiche - *Quercus robur L.* mit den färbenden Hauptinhaltsstoffen aus der Gruppe der Gallotannine und Ellagentannine (Glykoside der Gallussäure beziehungsweise der Ellagsäure; C.I. 75270) erhältlich.

In einer bevorzugten Ausführungsform enthält das erfindungsgemäße das Mittel als natürlichen Farbstoff mindestens eine Verbindung, ausgewählt aus Indigo, Lawson, Indigocarmin, Alizarin, Ruberythinsäure, Carotin, Curcumin, Quercetin, Rutin, Catechin, Lapachol, Juglon und/oder Riboflavin.

Die natürlichen Farbstoffe bzw. die Extrakte natürlichen Ursprungs, die die Farbstoffe enthalten, sind vorzugsweise zu 0,005 bis 25 Gew.-%, bevorzugt zu 0,05 bis 15 Gew.-%, jeweils bezogen auf das Gesamtgewicht des anwendungsbereiten Mittels, enthalten.

Ein zweiter wesentlicher Inhaltsstoff des Mittels des ersten Erfindungsgegenstands ist zweiwertiges Metall-Salz. Dadurch wird insbesondere die Haltbarkeit und damit die Echtheit der Färbung verbessert. Erfindungsgemäß ist darunter ein Metallsalz zu verstehen, dessen kationischer Anteil ein Metallkation mit der Oxidationsstufe "zwei" umfasst. Bevorzugte Metallsalze sind daher solche, deren Metall eine stabiles zweiwertiges Metallkation ausbilden kann. Hierzu zählen im Wesentlichen Erdalkalimetalle (im Periodensystem Gruppe IIA gemäß CAS-Nomenklatur bzw. Gruppe 2 gemäß IUPAC-Nomenklatur), Metalle der Gruppe IIB gemäß CAS-Nomenklatur (IUPAC: Gruppe 12) sowie geeignete Metalle der Gruppen VIIB und VIIIB gemäß CAS-Nomenklatur (IUPAC: Gruppen 7, 8, 9, und 10).

Eine Ausführungsform des ersten Erfindungsgegenstands ist daher ein Mittel, welches dadurch gekennzeichnet ist, dass das zweiwertige Metall-Salz ausgewählt ist zweiwertigen Metallkationen von Eisen, Magnesium, Calcium, Zink und Mangan.

Als anionisches Gegenion eignen sich erfindungsgemäß solche Anionen, die physiologisch verträglich sind. Hierzu zählen insbesondere Halogenide sowie die Anionen von kurzkettigen Carbonsäuren, wie Essigsäure, Weinsäure, Äpfelsäure oder Citronensäure, von Fettsäuren, wie Stearin- oder Palmitinsäure, von Aminosäuren, wie Asparaginsäure oder Glutaminsäure, oder von Fruchtsäuren, wie Gluconsäuren.

Eine besondere Ausführungsform des ersten Erfindungsgegenstands ist daher ein Mittel, welches dadurch gekennzeichnet ist, dass das zweiwertige Metall-Salz ausgewählt ist aus der Gruppe, die gebildet wird aus Eisen(II)chlorid, Eisen(II)gluconat, Eisen(II)citrat, Eisen(II)palmitat, Eisen(II)-aspartat, Calciumchlorid, Calciumaspartat, Calciumgluconat, Calciumcitrat, Calciumpalmitat, Magnesiumchlorid, Magnesiumaspartat, Magnesiumgluconat, Magnesiumcitrat, Magnesiumpalmitat, Zink(II)chlorid, Zink(II)aspartat, Zink(II)gluconat, Zink(II)citrat, Zink(II)palmitat, Mangan(II)-chlorid, Mangan(II)aspartat, Mangan(II)gluconat, Mangan(II)citrat und Mangan(II)palmitat.

Das zweiwertige Metall-Salz ist bevorzugt in einem Anteil von 0,005 bis 25 Gew.-%, bevorzugt zu 0,05 bis 15 Gew.-%, insbesondere 0,1 bis 10 Gew.-% und besonders bevorzugt von 0,2 bis 5 Gew.-%, jeweils bezogen auf das Gesamtgewicht des anwendungsbereiten Mittels, enthalten.

Als dritten wesentlichen Inhaltsstoff enthält das erfindungsgemäße Mittels mindestens ein amphoteres Polymer.

Als amphotere Polymere sind solche Polymere zu verstehen, die wenigstens ein quartäres Stickstoffatom, beispielsweise in Form einer Ammoniumgruppe, enthalten. Geeignete amphotere Polymere sind beispielsweise
- Copolymere von Diallyldimethylammoniumchlorid.

Eine bevorzugte Ausführungsform des ersten Erfindungsgegenstands ist ein Mittel, welches dadurch gekennzeichnet ist, dass das Mittel amphoteres Polymer mindestens ein Copolymer von Diallyldimethylammoniumchlorid enthält.

In den Copolymeren von Diallyldimethylammoniumchlorid werden anionische Monomere eingesetzt.

In einer besonders bevorzugten Ausführungsform ist das Copolymer von Diallyldimethylammoniumchlorid ausgewählt ist aus , -7), Diallyldimethylammoniumchlorid-Acrylsäure-Copolymer (Handelsname Merquat 280 bzw. Merquat 281; Polyquaternium-22), Diallyldimethylammoniumchlorid-Acrylsäureamid-Acrylsäure-Copolymer (Handelsname Merquat Plus 3330; Polyquaternium-39) .

Es ist bevorzugt, wenn dass kationische und/oder amphotere Polymer in einem Anteil von 0,01 bis 15 Gew.-%, bevorzugt 0,1 bis 10 Gew.-%, jeweils bezogen auf das Gesamtgewicht des anwendungsbereiten Mittels, im erfindungsgemäßen Mittel enthalten ist.

Ferner können die erfindungsgemäß eingesetzten Mittel weitere Wirk-, Hilfs- und Zusatzstoffe, wie beispielsweise
- anionische Tenside, wie Alkyl(ether)sulfate, Alkylethercarbonsäuren und Fettsäuren,
- zwitterionische Tenside, wie Cocamidopropyl Betaine,
- ampholytische Tenside, wie Disodium Cocoamphodipropionate und Disodium Cocoamphodiacetate,
- nichtionische Tenside, wie ethoxylierte Fettalkohole bzw. Fettsäuren und Alkylpolyglucoside
- kationische Tenside, wie Alkyltrimethylammoniumchloride, Dialkyldimethylammoniumchloride, Quaternium-27 und Quaternium-83 oder Esterquats.
- nichtionische oder anionische Polymere
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe und Pigmente zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Konsistenzgeber wie Zuckerester, Polyolester oder Polyolalkylether,
- pH-Stellmittel, wie Ammoniak, Monoethanolamin oder Citronensäure,
- Öle, insbesondere pflanzliche Öle, wie Olivenöl, Mandelöl, Macadamianussöl, Leinsamenöl, Sonnenblumenöl, Jojobaöl, Traubenkernöl, Aprikosenkernöl oder Orangenöl,
- Fette und Wachse wie Fettalkohole, Bienenwachs, Montanwachs und Paraffine,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Der Fachmann kennt diese Wirk-, Hilfs- und Zusatzstoffe und weiß sie entsprechend der gewünschten Eigenschaften des Mittels zuzusetzen.

Um Unverträglichkeiten zwischen den einzelnen Inhaltsstoffen im Mittel zu vermeiden, ist es vorteilhaft, wenn der natürliche Farbstoff und das zweiwertige Metall-Salz erst unmittelbar vor der Anwendung miteinander in Kontakt kommen. Eine getrennte Verpackung der beiden Inhaltsstoffe ist daher besonders bevorzugt.

Ein zweiter Gegenstand der vorliegenden Erfindung ist daher eine Mehrkomponentenverpackungseinheit (Kit-of-Parts) zur Färbung keratinischer Fasern, umfassend mindestens zwei voneinander getrennt konfektionierte Zubereitungen (I) und (II), welche dadurch gekennzeichnet ist, dass
a) die Zubereitung (I) in einem kosmetisch verträglichen Träger mindestens einen natürlichen Farbstoff, ausgewählt aus Alkaloid-Farbstoffen, Anthrachinon-Farbstoffen, Betalainen, Carotinoiden, Cathechinen, Curcumidoiden, Flavinen, Flavonoiden, indigoiden Farbstoffen, Naphthochinonen und weiteren phenolischen Farbstoffen und weiterhin mindestens ein amphoteres Polymer enthält, und dass
b) die Zubereitung (II) in einem kosmetisch verträglichen mindestens ein zweiwertiges Metall-Salz enthält.

Eine Mehrkomponentenverpackungseinheit umfasst erfindungsgemäß mehrere Einzelkomponenten, die getrennt voneinander konfektioniert sind, sowie eine gemeinsame Verpackung für diese Komponenten, wie zum Beispiel eine Faltschachtel. Darin werden die Komponenten jeweils getrennt in verschiedenen Containern bereitgestellt. Unter Container wird im Rahmen der vorliegenden Erfindung eine Umhüllung verstanden, die in Form einer gegebenenfalls wiederverschließbaren Flasche, einer Tube, einer Dose, eines Tütchens, eines Sachets oder einer ähnlichen Umhüllung vorliegt. Dem Umhüllungsmaterial sind erfindungsgemäß keine Grenzen gesetzt. Bevorzugt handelt es sich jedoch dabei um Umhüllungen aus Glas oder Kunststoff.

Darüber hinaus kann die Verpackungseinheit Applikationshilfen, wie Kämme, Bürsten oder Pinsel, persönliche Schutzkleidung, insbesondere Ein-Weg-Handschuhe, sowie eine Gebrauchsanleitung umfassen.

Es ist erfindungsgemäß vorteilhaft, wenn die beiden Zubereitungen (I) und (II) erst unmittelbar vor der Anwendung miteinander vermischt werden.

Ein dritter Gegenstand der vorliegenden Erfindung ist daher ein Verfahren zur Färbung menschlicher Haare, dadurch gekennzeichnet, dass

in einem ersten Schritt eine Zubereitung (I), die in einem kosmetisch verträglichen Träger mindestens einen natürlichen Farbstoff, ausgewählt aus Alkaloid-Farbstoffen, Anthrachinon-Farbstoffen, Betalainen, Carotinoiden, Cathechinen, Curcumidoiden, Flavinen, Flavonoiden, indigoiden Farbstoffen, Naphthochinonen und weiteren phenolischen Farbstoffen und weiterhin mindestens ein amphoteres Polymer enthält, und eine Zubereitung (II), die in einem kosmetisch verträglichen mindestens ein zweiwertiges Metall-Salz enthält, zu einer Anwendungsmischung innig miteinander vermischt werden, und
in einem zweiten Schritt die resultierende Anwendungszubereitung für eine Einwirkzeit von 10 bis 45 min auf das Haar aufgetragen wird und anschließend mit Wasser ausgewaschen wird.

Bevorzugt wird der zweite Schritt innerhalb weniger Minuten, besonders bevorzugt unmittelbar nach dem ersten Schritt ausgeführt.

Die Anwendungstemperaturen während des zweiten Schritts liegen bevorzugt in einem Bereich zwischen 15 und 45 °C. Gegebenenfalls kann während der Einwirkzeit zusätzlich Wärme zugeführt werden, beispielsweise mit eine Haube oder einem Heißluftgebläse. Die Einwirkungszeit beträgt 10 0 bis 45 min, bevorzugt 15 bis 35 min.

Es zeigt sich, dass die Anwendung des erfindungsgemäßen Mittels des ersten Erfindungsgegenstands zu verringerter Haarschädigung führt.

Ein vierter Gegenstand der vorliegenden Erfindung ist schließlich die Verwendung eines Mittels des ersten Erfindungsgegenstands zur Verringerung der Haarschädigung bei der Färbung menschlicher Haare.

Für bevorzugte Ausführungsformen des zweiten, dritten und vierten Erfindungsgegenstands gelten mutatis mutandis die Ausführungsformen des ersten Erfindungsgegenstands.

### Beispiele

Das Färbemittel "Permanent Hair Coloring Kit 8BC Intense light copper blonde" der Firma Advanced Cosmetic Technologies, umfassend eine Färbezubereitung und eine Salzzubereitung, wurde entsprechend Tabelle 1 mit verschiedenen Polymeren versetzt.

**Tabelle 1.**

| | A (Vergleich) | B | C |
|---|---|---|---|
| Färbezubereitung 8BC Intense light copper blonde | - | Merquat 100* (10 Gew.-%) | Merquat 280** (10 Gew.-%) |

| | | | |
|---|---|---|---|
| *Merquat 100 Polyquaternium-6 (ca. 40 Gew.-%, wässrig) **Merquat 280 Polyquaternium-22 (ca. 40 Gew.-%, wässrig) | | | |

### Färbezubereitung 8BC Intense light copper blonde (INCI-Inhaltsangabe):

Aqua, Glycerin, Lawsonia Inermis, Rubia Cordifolia Root Extract, Indigofera Tinctoria Extract, C.I. 58000 from Rubia Tinctorum Root Extract, C.I. 73015 from Indigofera Tinctoria Extract, Beetroot Extract, Quercetin, Rutin, Decyl Glucoside, Haematoxylon Campechian Wood Extract, Haematoxylum Braziletto Extract, Curcuma Longa (Turmeric) Root Extract, Astaxanthin, Crocetin (C.I. 75100), C.I. 75810 From Chlorophyllin-Copper Complex, Iron-Chlorophyllin Complex, Annatto (C.I. 75120), Gardenia Jasminoides Fruit Extract, Emblica Officinalis Fruit Extract, Saffron Crocus (Crocus Sativus) Extract, Citric Acid, Lactic Acid, Benzyl Alcohol, Green Tea Extract, Anthacyanins, Pomegranate Extract, Sweet Almond Oil, 100% Natural Essential Oils.

### Salzzubereitung (INCI-Inhaltsangabe):

Aqua, Glycerin, Xanthan Gum, Magnesium Gluconate, Hydroxyethylcellulose, Chondrus Crispus (Carrageenan), Magnesium Aspartate, Magnesium Citrate, Citric Acid, Isopropyl Alcohol, Ferrous Aspartate, Copper Gluconate, Zinc Gluconate, Zinc Aspartate, Copper Aspartate, Calcium Gluconate, Ferric Ammonium Citrate, Ferrous Gluconate, Calcium Aspartate, Calcium Chloride, Lactic Acid, Benzyl Alcohol, Sweet Almond Oil, 100% Natural Essential Oils.

### Wirknachweise - Naßkämmarbeit

Die Färbezubereitungen gemäß Tabelle 1 wurden jeweils in gleichen Gewichtsteilen mit einer Salzubereitung vermischt. Je 4 g dieser Anwendungszubereitung wurden auf 1 g Haarsträhne (hellbraun, Kerling ENH 6/0) aufgetragen und für 30 min bei 32°C einwirken gelassen. Die Haarsträhnen wurden anschließend mit Leitungswasser (32°C) ausgespült. Pro Anwendungszubereitung wurden jeweils 10 Strähnen behandelt und untersucht.

Vor der Anwendung der Testformulierungen wurden die wassernassen Strähnen 10x automatisch mit Hartgummikämmen (Hercules Sägemann) gekämmt und die aufzuwendende Arbeit bestimmt. Nach Anwendung der Mittel wurden die Strähnen mit Wasser für 2 min gespült und die Messungen wiederholt. Es wurden jeweils 10 Haarsträhnen behandelt und gemessen. Als Wert für die Kämmarbeit wurde jeweils das arithmetische Mittel gebildet. Folgende Ergebnisse wurden erhalten:

| | Naßkämmarbeit |
|---|---|
| unbehandelt | - |
| A (Vergleich) | **-19%** |
| B (+ Merquat 100) | **- 43 %** |
| C (+ Merquat 280) | **- 52 %** |

Die Ergebnisse zeigen eine deutliche Verbesserung der Naßkämmbarkeit in den erfindungsgemäßen Mitteln. "C".

## Patentansprüche

1. Mittel zur Färbung menschlicher Haare, enthaltend in einem kosmetisch verträglichen Träger mindestens einen natürlichen Farbstoff, ausgewählt aus Alkaloid-Farbstoffen, Anthrachinon-Farbstoffen, Betalainen, Carotinoiden, Cathechinen, Curcumidoiden, Flavinen, Flavonoiden, indigoiden Farbstoffen, Naphthochinonen und weiteren phenolischen Farbstoffen sowie mindestens ein zweiwertiges Metall-Salz, **dadurch gekennzeichnet, dass** das Mittel weiterhin mindestens ein amphoteres Polymer enthält.

2. Mittel nach Anspruch 1, **dadurch gekennzeichnet, dass** das zweiwertige Metall-Salz ausgewählt ist zweiwertigen Metallkationen von Eisen, Magnesium, Calcium, Zink und Mangan.

3. Mittel nach einem der Ansprüche 1 oder 2, **dadurch gekennzeichnet, dass** das zweiwertige Metall-Salz ausgewählt ist aus der Gruppe, gebildet aus Eisen(II)chlorid, Eisen(II)gluconat, Eisen(II)citrat, Eisen(II)palmitat, Eisen(II)aspartat, Calciumchlorid, Calciumaspartat, Calciumgluconat, Calciumcitrat, Calciumpalmitat, Magnesiumchlorid, Magnesiumaspartat, Magnesiumgluconat, Magnesiumcitrat, Magnesiumpalmitat, Zink(II)chlorid, Zink(II)aspartat, Zink(II)gluconat, Zink(II)citrat, Zink(II)palmitat, Mangan(II)chlorid, Mangan(II)aspartat, Mangan(II)gluconat, Mangan(II)citrat und Mangan(II)palmitat.

4. Mittel nach einem der Ansprüche 1 bis 3, **dadurch gekennzeichnet, dass** das Mittel als natürlichen Farbstoff mindestens eine Verbindung, ausgewählt aus Indigo, Lawson, Indigocarmin, Alizarin, Ruberythinsäure, Carotin, Curcumin, Quercetin, Rutin, Chlorophyll A/B, Catechin, Lapachol, Juglon und/oder Riboflavin, enthält.

5. Mittel nach einem der Ansprüche 1 bis 4, **dadurch gekennzeichnet, dass** das Mittel als amphoteres Polymer mindestens ein Copolymer von Diallyldimethylammoniumchlorid enthält.

6. Mittel nach einem der Ansprüche 1 bis 5, **dadurch gekennzeichnet, dass** das Copolymer von Diallyldimethylammoniumchlorid ausgewählt ist aus Diallyldimethylammoniumchlorid-Acrylsäure-Copolymer, Diallyldimethylammoniumchlorid-Acrylsäureamid-Acrylsäure-Copolymer

7. Mittel nach einem der Ansprüche 1 bis 6, **dadurch gekennzeichnet, dass** das amphotere Polymer in einem Anteil von 0,01 bis 15 Gew.-%, bevorzugt 0,1 bis 10 Gew.%, jeweils bezogen auf das Gesamtgewicht des anwendungsbereiten Mittels, enthalten ist.

8. Mehrkomponentenverpackungseinheit zur Färbung keratinischer Fasern, umfassend mindestens zwei voneinander getrennt konfektionierte Zubereitungen, **dadurch gekennzeichnet, dass**
die Zubereitung (I) in einem kosmetisch verträglichen Träger mindestens einen natürlichen Farbstoff, ausgewählt aus Alkaloid-Farbstoffen, Anthrachinon-Farbstoffen, Betalainen, Carotinoiden, Cathechinen, Curcumidoiden, Flavinen, Flavonoiden, indigoiden Farbstoffen, Naphthochinonen und weiteren phenolischen Farbstoffen und weiterhin mindestens ein amphoteres Polymer enthält, und dass
die Zubereitung (II) in einem kosmetisch verträglichen mindestens ein zweiwertiges Metall-Salz enthält.

9. Verfahren zur Färbung menschlicher Haare, **dadurch gekennzeichnet, dass** in einem ersten Schritt eine Zubereitung (I), die in einem kosmetisch verträglichen Träger mindestens einen natürlichen Farbstoff, ausgewählt aus Alkaloid-Farbstoffen, Anthrachinon-Farbstoffen, Betalainen, Carotinoiden, Cathechinen, Curcumidoiden, Flavinen, Flavonoiden, indigoiden Farbstoffen, Naphthochinonen und weiteren phenolischen Farbstoffen und weiterhin mindestens ein amphoteres Polymer enthält, und eine Zubereitung (II), die in einem kosmetisch verträglichen mindestens ein zweiwertiges Metall-Salz enthält, zu einer Anwendungsmischung innig miteinander vermischt werden, und
in einem zweiten Schritt die resultierende Anwendungszubereitung für eine Einwirkzeit von 10 bis 45 min auf das Haar aufgetragen wird und anschließend mit Wasser ausgewaschen wird.

10. Verwendung eines Mittels nach einem der Ansprüche 1 bis 7, zur Verringerung der Haarschädigung bei der Färbung menschlicher Haare.

## Claims

1. Agent for dyeing human hair, containing in a cosmetically acceptable carrier at least one natural dye selected from alkaloid dyes, anthraquinone dyes, betalaines, carotenoids, catechins, curcumins, flavins, flavonoids, indigoid dyes, naphthoquinones, and further phenolic dyes, and at least one divalent metal salt, **characterized in that** the agent further contains at least one amphoteric polymer.

2. Agent according to Claim 1, **characterized in that** the divalent metal salt is selected from divalent metal cations of iron, magnesium, calcium, zinc, and manganese.

3. Agent according to one of Claims 1 or 2, **characterized in that** the divalent metal salt is selected from the group comprising iron(li) chloride, iron(II) gluconate, iron(II) citrate, iron(II) palmitate, iron(II) aspartate, calcium chloride, calcium aspartate, calcium gluconate, calcium citrate, calcium palmitate, magnesium chloride, magnesium aspartate, magnesium gluconate, magnesium citrate, magnesium palmitate, zinc(II) chloride, zinc(II) aspartate, zinc(II) gluconate, zinc(II) citrate, zinc(II) palmitate, manganese(II) chloride, manganese(II) aspartate, manganese(II) gluconate, manganese(II) citrate, and manganese(II) palmitate.

4. Agent according to one of Claims 1 to 3, **characterized in that** the agent contains at least one compound selected from indigo, lawsone, indigo carmine, alizarin, ruberythric acid, carotene, curcumin, quercetin, rutin, chlorophyll A/B, catechin, lapachol, juglone, and/or riboflavin as natural dye.

5. Agent according to one of Claims 1 to 4, **characterized in that** the agent contains at least one copolymer of diallyldimethylammonium chloride as amphoteric polymer.

6. Agent according to one of Claims 1 to 5, **characterized in that** the copolymer of diallyldimethylammonium chloride is selected from diallyldimethylammonium chloride-acrylic acid copolymer or diallyldimethylammonium chloride-acrylic acid amide-acrylic acid copolymer.

7. Agent according to one of Claims 1 to 6, **characterized in that** the amphoteric polymer is contained in a proportion of 0.01 to 15% by weight, preferably 0.1 to 10% by weight, in each case based on the total weight of the ready-to-use agent.

8. Multicomponent packaging unit for coloring keratinic fibers, comprising at least two preparations which are prepared separately, **characterized in that**
preparation (I) contains in a cosmetically acceptable carrier at least one natural dye selected from alkaloid dyes, anthraquinone dyes, betalaines, carotenoids, catechins, curcumins, flavins, flavonoids, indigoid dyes, naphthoquinones, and further phenolic dyes, and also at least one amphoteric polymer, and that
preparation (II) contains in a cosmetically acceptable carrier at least one divalent metal salt.

9. Method for dyeing human hair, **characterized in that** in a first step a preparation (I) which in a cosmetically acceptable carrier contains at least one natural dye selected from alkaloid dyes, anthraquinone dyes, betalaines, carotenoids, catechins, curcumins, flavins, flavonoids, indigoid dyes, naphthoquinones, and further phenolic dyes, and also at least one amphoteric polymer, and a preparation (II) which in a cosmetically acceptable carrier contains at least one divalent metal salt are intimately mixed together to form an application mixture, and
in a second step the resulting application preparation is applied to the hair for an exposure time of 10 to 45 min and subsequently washed out with water.

10. Use of an agent according to one of Claims 1 to 7 for reducing hair damage during the dyeing of human hair.

## Revendications

1. Agent de teinture de cheveux humains, contenant dans un support cosmétiquement acceptable au moins un colorant naturel choisi parmi les colorants alcaloïdiques, les colorants anthraquinoniques, les bétalaïnes, les caroténoïdes, les cathéchines, les curcumidoïdes, les flavines, les flavonoïdes, les colorants indigoïdes, les naphtoquinones et autres colorants phénoliques ainsi qu'au moins un sel métallique divalent, **caractérisé en ce que** l'agent contient en outre au moins un polymère amphotère.

2. Agent selon la revendication 1, **caractérisé en ce que** le sel métallique divalent est choisi parmi les cations métalliques divalents du fer, du magnésium, du calcium, du zinc et du manganèse.

3. Agent selon l'une quelconque des revendications 1 ou 2, **caractérisé en ce que** le sel métallique divalent est choisi dans le groupe formé par le chlorure ferreux, le gluconate ferreux, le citrate ferreux, le palmitate ferreux, l'aspartate ferreux, le chlorure de calcium, l'aspartate de calcium, le gluconate de calcium, le citrate de calcium, le palmitate de calcium, le chlorure de magnésium, l'aspartate de magnésium, le gluconate de magnésium, le citrate de magnésium, le palmitate de magnésium, le chlorure de zinc(II), l'aspartate de zinc(II), le gluconate de zinc(II), le citrate de zinc(II), le palmitate de zinc(II), le chlorure de manganèse(II), l'aspartate de manganèse(II), le gluconate de manganèse(II), le citrate de manganèse(II) et le palmitate de manganèse(II).

4. Agent selon l'une des revendications 1 à 3, **caractérisé en ce que** l'agent contient comme colorant naturel au moins un composé choisi parmi l'indigo, le Lawson, le carmin d'indigo, l'alizarine, l'acide rubérythinique, le carotène, la curcumine, la quercétine, la rutine, la chlorophylle A/B, la catéchine, le lapachol, la juglone et / ou la riboflavine.

5. Agent selon l'une des revendications 1 à 4, **caractérisé en ce que** l'agent contient comme polymère amphotère au moins un copolymère du chlorure de diallyldiméthylammonium.

6. Agent selon l'une des revendications 1 à 5, **caractérisé en ce que** le copolymère du chlorure de diallyldiméthylammonium est choisi parmi le copolymère chlorure de diallyldiméthylammonium-acide acrylique et le copolymère chlorure de diallyldiméthylammoniumacrylamide-acide acrylique.

7. Agent selon l'une des revendications 1 à 6, **caractérisé en ce que** le polymère amphotère est contenu dans une proportion de 0,01 à 15% en poids, de préférence 0,1 à 10% en poids, à chaque fois sur la base du poids total de l'agent prêt à l'emploi.

8. Unité de conditionnement multi-composants pour la teinture de fibres kératiniques, comprenant au moins deux préparations confectionnées séparément, **caractérisée en ce que** la préparation (I) contient dans un support cosmétiquement acceptable au moins un colorant naturel choisi parmi les colorants alcaloïdiques, les colorants anthraquinoniques, les bétalaïnes, les caroténoïdes, les cathéchines, les curcumidoïdes, les flavines, les flavonoïdes, les colorants indigoïdes, les naphtoquinones et autres colorants phénoliques et en outre au moins un polymère amphotère, et **en ce que**
la préparation (II) contient dans un support cosmétiquement acceptable au moins un sel métallique divalent.

9. Procédé de teinture de cheveux humains, **caractérisé en ce que** dans une première étape une préparation (I), qui contient dans un support cosmétiquement acceptable au moins un colorant naturel choisi parmi les colorants alcaloïdiquees, les colorants anthraquinoniques, les bétalaïnes, les caroténoïdes, les cathéchines, les curcumidoïdes, les flavines, les flavonoïdes, les colorants indigoïdes, les naphtoquinones et autres colorants phénoliques et en outre au moins un polymère amphotère, et une préparation (II) qui contient dans un support cosmétiquement acceptable au moins un sel métallique divalent, sont mélangées intimement l'une à l'autre pour donner un mélange d'application, et
et dans une seconde étape, la préparation d'application résultante est appliquée sur les cheveux pendant une durée d'action de 10 à 45 min puis est éliminée par lavage avec de l'eau.

10. Utilisation d'un agent selon l'une des revendications 1 à 7, pour réduire l'endommagement des cheveux lors de la teinture de cheveux humains.
